# EUROPÄISCHE PATENTSCHRIFT

(11) **EP 1 797 191 B1**
(45) Veröffentlichungstag und Bekanntmachung des Hinweises auf die Patenterteilung: **02.04.2008**
(21) Anmeldenummer: 05787273.1
(22) Anmeldetag: 22.09.2005
(51) Int. Cl.: C12P 7/04

(54) **VERFAHREN ZUR HERSTELLUNG VON OPTISCH AKTIVEN 2-METHYLALKAN-1-OLEN AUS DEN ENTSPRECHENDEN 2-METHYLALK-2-EN-1-ALEN, UMFASSEND EINEN SCHRITT DER ENANTIOSELEKTIVEN ACYLIERUNG ZWECKS ANREICHERUNG EINES ENATIOMERS**
PROESS FOR PRODUCING AN OPTICALLY ACTIVE 2-METHYL-ALKANE-1-OLS FROM THE CORRESPONDING 2-METHYLALK-2-EN-1-ALS, COMPRISING A STEP OF ENANTIOSELECTIVE ACYLATION TO ENRICH ONE ENANTIOMER
PROCEDE DE PREPARATION DES 2-METHYL-ALCAN-1-OLS OPTIQUEMENT ACTIFS A PARTIR DES 2-METHYLALC-2-EN-1-ALS CORRESPONDANTS, COMPRENANT UNE ETAPE D'ACYLATION ENANTIOSELECTIVE POUR ENRICHIR D'UN ENANTIOMERE

(30) Priorität: 29.09.2004 DE 102004047836
(43) Veröffentlichungstag der Anmeldung: 20.06.2007
(73) Patentinhaber: BASF SE, 67056 Ludwigshafen (DE)
(72) Erfinder: JÄKEL, Christoph, 67117 Limburgerhof (DE); HEYDRICH, Gunnar, 67117 Limburgerhof (DE); STÜRMER, Rainer, 67127 Rödersheim-Gronau (DE); PACIELLO, Rocco, 67098 Bad Dürkheim (DE)
(86) Internationale Anmeldenummer: PCT/EP2005/010240
(87) Internationale Veröffentlichungsnummer: WO 2006/034812

(56) Entgegenhaltungen:
- EP-A- 0 492 401
- EP-A- 0 529 698
- BIANCHI, D. ET AL.: "Anhydrides as Acylating Agents in Lipase-Catalyzed Stereoselective Esterification of Racemic Alcohols" JOURNAL OF ORGANIC CHEMISTRY, Bd. 53, 1988, Seiten 5531-5534, XP002274382
- BARTH, S. & EFFENBERGER, F.: "Lipase-Catalyzed Resolution of Racemic 2-Alkyl Substituted 1-Alkanols" TETRAHEDRON: ASYMMETRY, Bd. 4, Nr. 5, 1993, Seiten 823-833, XP002380147 in der Anmeldung erwähnt
- HÖGBERG, H.E.: "Approaches to 2-methyl-1-alkanols of high enantiomeric purities via enzyme mediated reactions" NATO ASI SERIES. SERIES C, Bd. 381, 1992, Seiten 399-410, XP008063921

## Beschreibung

Die vorliegende Erfindung betrifft ein neues Verfahren zur Herstellung von optisch aktivem 2-Methylalkan-1-ol ausgehend von 2-Alkylpent-2-enal.

### Stand der Technik

Die Herstellung von optisch aktivem 2-Methylpentan-1-ol ("Methylpentanol") ist in der Literatur gemäß verschiedener Verfahren vorbeschrieben. So erhalten z. B. M. A. Jermyn et al. (Aust. J. Chem. 1967, 20, 2283-2284) R-Methylpentanol durch Hydrolyse des p-Toluensulphonats des L-Valin-(R)-2-methyl-pentyl ester. Oppolzer et al. (Helv. Chim. Acta 1985, 68, 212-215) erhalten R-Methylpentanol über diastereoselektive Ester-Enolat-Alkylierung eines chiralen Sultams. Danishefsky et al. (J. Org. Chem. 1986, 51, 5032-5036) erhalten R-2-Methylpentanol über diastereoselektive Imid-Enolat-Alkylierung eines chiralen Oxazolidinons. Effenberger et al. (Tetrahedron: Asymmetry 1993, 4, 823-833) erhalten R-Methylpentanol aus racemischem Methylpentanol durch Lipase-katalysierte enantioselektive Acylierung. Die oben beschriebenen Verfahren erzeugen optisch aktives Methylpentanol aufgrund teurer Einsatzstoffe, vielstufiger Synthese, mangelnden Ausbeuten oder hohem Reinigungsaufwand in einer wirtschaftlich unbefriedigenden Weise.

### Aufgabenstellung

Es bestand daher die Aufgabe, ein Verfahren zur Herstellung von optisch aktivem 2-Methylalkan-1-ol bereitzustellen, dass die oben geschilderten Nachteile vollständig oder zumindest teilweise vermeidet.

### Beschreibung der Erfindung

Gegenstand der Erfindung ist ein Verfahren zur Herstellung von optisch aktivem 2-Methylalkan-1-ol der allgemeinen Formel (III) umfassend folgende Schritte:
(i) carbonylselektive Reduktion von 2-Methylalk-2-en-1-al der allgemeinen Formel (I) zu 2-Methylalk-2-en-1-ol der allgemeinen Formel (II),
(ii) enantioselektive Hydrierung von 2-Methylalk-2-en-1-ol zu der allgemeinen Formel (iii),
(iii) Erhöhung der optischen Ausbeute des in Schritt (ii) erhaltenen optisch aktiven 2-Methylalkan-1-ol (III) durch eine Lipase-katalysierte Acylierungsreaktion, 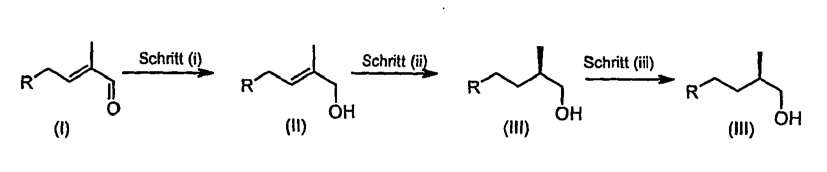
wobei der Rest R die Bedeutung C₁-C₁₀- Alkyl besitzt.

Im oben gezeigten Formelschema ist nur ein Enantiomer (III) eingezeichnet. Es wird jedoch darauf hingewiesen, dass das erfindungsgemässe Verfahren auch die Herstellung des jeweils anderen -hier nicht gezeichneten - Enantiomeren (III) mitumfasst. Das jeweils gewünschte Enantiomer (III) kann durch Auswahl des entsprechenden Katalysatorsystems in Schritt (ii) erhalten werden.

In den oben gezeigten Formelbildern (I) bis (III) hat der Rest R die Bedeutung C₁-C₁₀-Alkyl, wobei der Alkylrest geradkettig oder verzweigt sein kann, insbesondere Methyl, Ethyl, n-Propyl und iso-Propyl, n-Butyl, iso-Butyl, tert.-Butyl, Pentyl, Hexyl, Heptyl, Oktyl, Nonyl und Decyl. Weitererhin umfasst diese Definition auch substituierte Alkyle, bei denen ein oder mehrere, bevorzugt ein bis drei Wasserstoffatome durch Reste wie, F, Cl, Br, J, NO₂, NH₂, NH(Alkyl), N(Alkyl)₂, OH, SH, CN, ersetzt sind.

Im folgenden werden die einzelnen Schritte des Verfahrens beschrieben:

### Schritt (i): Carbonyl-selektive Reduktion

Die carbonylselektive Reduktion von 2-Methylalk-2-en-1-al zu 2-Methylalk-2-en-1-ol kann durch verschiedene dem Fachmann bekannte Verfahren durchgeführt werden. Beispiele solcher Verfahren umfassen hydridübertragende Reagenzien oder Katalysatoren wie z. B. Hauptgruppenelementhydride oder Übergangsmetallkomplexe, die als Katalysatoren wirken können, Transferhydrierungen, Reduktionen mit Metallen oder niedervalenten Metallsalzen, Diimin-Reduktionen oder Hydrierungen. Eine Zusammenstellung solcher Verfahren ist beispielsweise in R. L. Larock, Comprehensive Organic Transformations, Wiley-VCH, New York, 1999 beschrieben.

Je nach Selektivität des in Schritt (i) verwendeten Katalysatorsystems und der gewählten Bedingungen kann in Schritt (i) bereits in geringen Mengen das überhydrierte Produkt (III) als Racemat anfallen. Da dieses Racemat (III) die in in Schritt (ii) vorgesehene enantioselektive Hydrierung der Doppelbindung in ihrer optischen Ausbeute verschlechtern würde, ist es ratsam, im Anschluss an Schritt (i) gegebenenfalls eine Reinigung des erzeugten 2-Methylalk-2-en-1-ol (II) durchzuführen, um evt. nicht reagiertes Ausgangsmaterial (I) oder überhydriertes racemisches (III) zu entfernen.

Eine solche Reinigung kann bevorzugt destillativ erfolgen, insbesondere kann zur Ausbeuteerhöhung der Zusatz einer höhersiedenden Komponente zu dem zu trennenden Reaktionsgemisch angebracht sein.

### Schritt (ii): enantioselektive Hydrierung

Die enantioselektive Hydrierung von 2-Methylalk-2-en-1-ol zu optisch aktivem 2-Methylalkan-1-ol kann mittles Übergangsmetallkomplexkatalysatoren, insbesonders solchen mit Übergangsmetallen der Gruppen 8-11 durchgeführt werden.

Übergangsmetallkomplexkatalysatoren können aus einem metallhaltigen Präkatalysator und einem Liganden gebildet werden.

Bevorzugt als Präkatalysator sind Komplexe enthaltend Ru, Rh, Ir, Pd, Pt, besonders bevorzugt als Präkatalysator sind Komplexe enthaltend Ru und Rh.

Solche bevorzugten Präkatalysatoren sind Metallkomplexe beispielsweise RhCl₃, Rh(OAc)₃, [Rh(cod)Cl]₂, Rh(CO)₂acac, [Rh(cod)OH]₂, [Rh(cod)OMe]₂, Rh₄(CO)₁₂, Rh₆(CO)₁₆ und RuCl₃, Ru(acac)₃, [Ru(benzen)Cl]₂, [Ru(cymen)l₂], Ru(methallyl)2(cod).

Die genannten und weitere geeignete Übergangsmetallverbindungen und - komplexe sind bekannt und in der Literatur beschrieben oder können vom Fachmann analog zu den bereits bekannten Verbindungen hergestellt werden.

Liganden sind Verbindungen, die P, As, Sb-haltig sind, außerdem Verbindungen die über ein Kohlenstoffatom an das Übergangsmetallkatalysatorkomplexfragment gebunden sind.

Besonders bevorzugt sind chirale P-haltige Liganden.

Besonders bevorzugt als Liganden sind phosphorhaltige Verbindungen mit der Fähigkeit Atropisomerie bezüglich zweier Aryl- bzw. Hetarylsysteme auszubilden gemäß der folgenden Darstellungen: 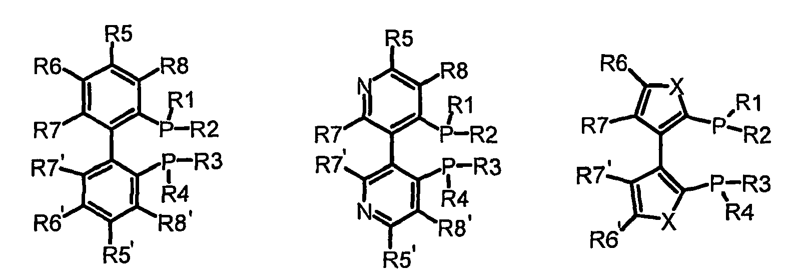 wobei die Reste folgende Bedeutung besitzen:
R1, R2 (gleich oder verschieden): subst. oder unsubst. Aryl, Heteroaryl, Alkyl, Cycloalkyl wobei die Subst. = H, Halogen, Alkyl, Alkoxy sein können
R3, R4 (gleich oder verschieden): subst. oder unsubst. Aryl, Heteroaryl, Alkyl, Cycloalkyl wobei die Subst. = H, Halogen, Alkyl, Alkoxy sein können
R5, R5', R6, R6' (gleich oder verschieden): mit R = H, Halogen, Alkyl, Aryl, Alkoxy, Amino, Thio
R7, R7' (gleich oder verschieden): mit R = H, Halogen, Alkyl, Aryl, Alkoxy, Amino, Thio
oder: R5, R5' (gleich oder verschieden): mit R = H, Halogen, Alkyl, Aryl, Alkoxy, Amino, Thio und R6 und R7, R6' und R7' formen einen oder mehrere Ringe, die weitere 1 oder 2 Doppelbindungen enthalten können (= annelierter, aromatischer Ring) und/oder Heteroatome (O, N, S) enthalten können
R8, R8' (gleich oder verschieden): mit R = H, Halogen, Alkyl, Aryl, Alkoxy, Amino, Thio
X, X' (gleich oder verschieden): mit X = S, O, NR9
   R9 = H, Alkyl, Aryl, Acyl, SO₂R10
   R10 = Aryl, Alkyl, Fluoroalkyl, CF₃

Besonders gut geeignete Liganden für das in Schritt (ii) verwendete Katalysatorsystem sind die folgenden aus der Literatur bekannten Liganden:.

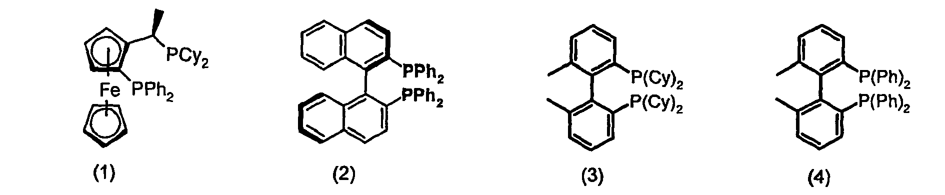

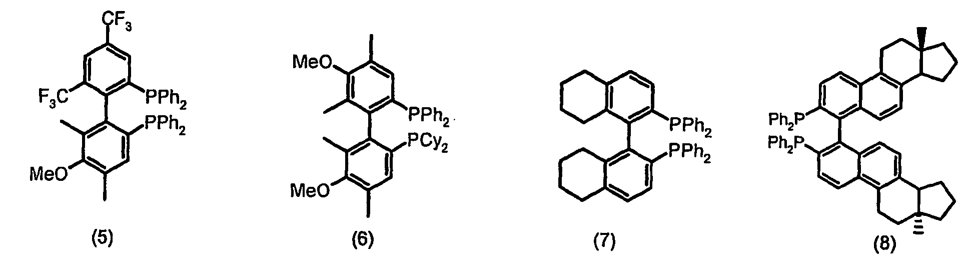

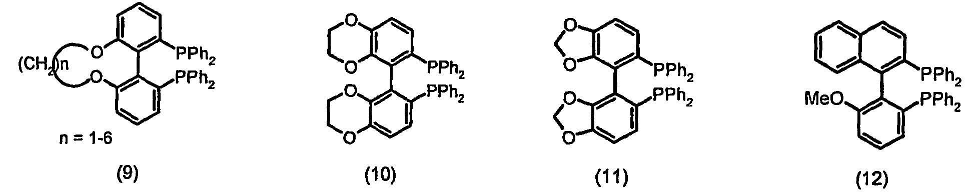

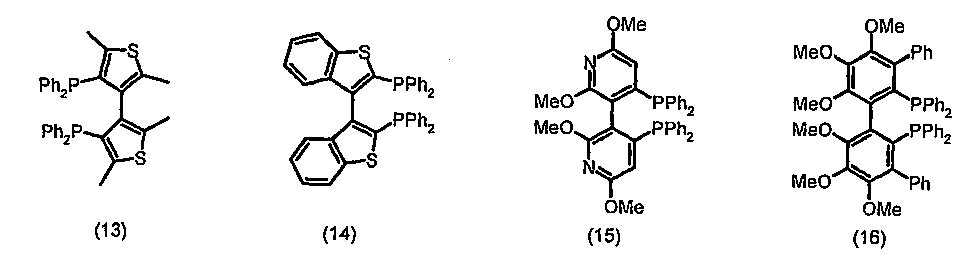

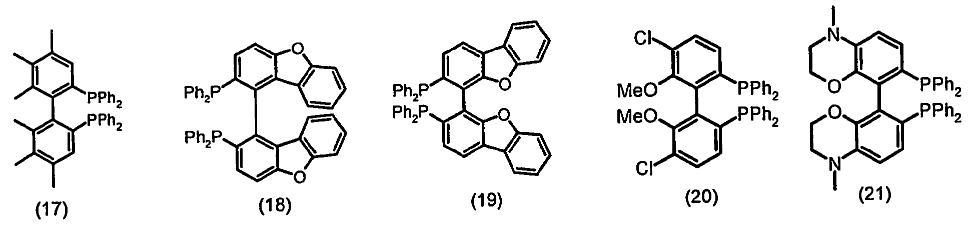

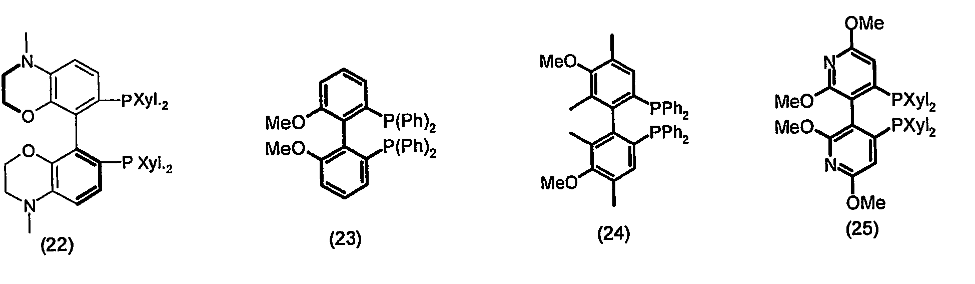

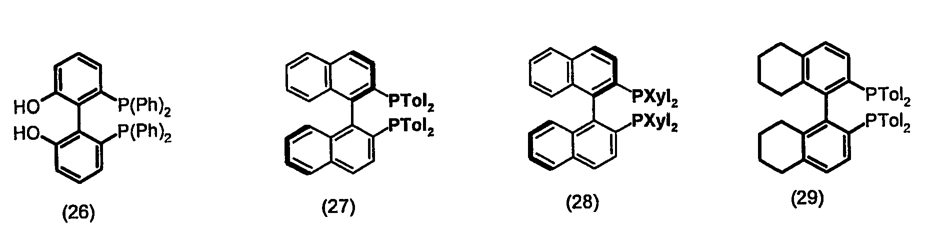

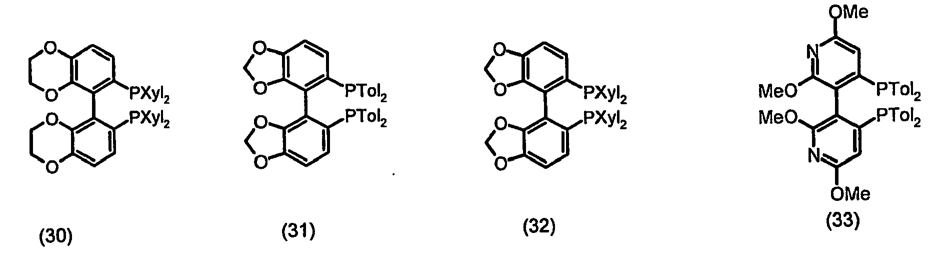

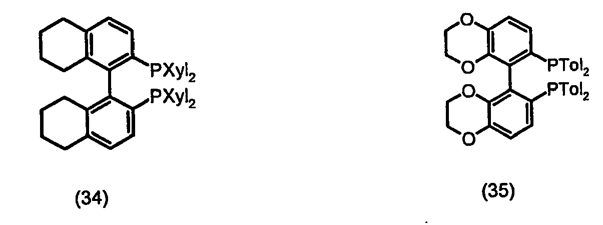

Die Herstellung der oben genannten Übergangsmetallkomplexkatalysatoren aus Präkatalysator und Ligand ist bekannt und in der Literatur beschrieben, z.B. H.U. Blaser, B. Pugin, F. Spindler in "Applied homogeneous and heterogeneous catalysis with organometallic compounds", Ed. B. Cornils, W.A. Herrmann, p. 992 ff. VCH Weinehim , 1996, ISBN , 3-527-29286-1

Die Reaktionstemperatur der enantioselektiven Hydrierung kann zwischen -10°C und 150°C liegen, bevorzugt zwischen 0 - 120°C, besonders bevorzugt zwischen 10 - 100°C.

Der Reaktionsdruck der enantioselektiven Hydrierung kann zwischen 0,1 bis 600 bar liegen, bevorzugt zwischen 50 und 250 bar.

Der für Schritt (ii) verwendete Katalysator wird üblicherweise in einem Verhältnis Kat:Substrat < 1:1000, bevorzugt < 1:5000 eingesetzt.

Die Reaktionszeit hängt üblicherweise von der Reaktionstemperatur sowie den bei der Reaktion herrschenden Druckverhältnissen und den verwendeten Katalysatormengen ab; üblicherweise beträgt sie zwischen 1 und 50 Stunden, bevorzugt, 5 bis 25 Stunden.

Die folgenden Lösungsmittel können bevorzugt verwendet werden: Methanol, Ethanol, i-Propanol, Propanol, Butanol, sec.-Butanol, tert.-Butanol, CH₂Cl₂, CHCl₃, Dichlorethan, EtOAc, THF, TBME, Et₂O, Bu₂O, Toluen, Xylen, Benzen, Alkane der allgemeinen Formel CₙH₂ₙ₊₂ mit n = 5-15 und Mischungen davon.

Besonders bevorzugt sind Methanol, Ethanol, Propanol, i-Propanol.

Schritt (iii): Erhöhung der optischen Ausbeute des in Schritt (ii) erhaltenen optisch aktiven 2-Methylalkan-1-ol (III) durch eine Lipase-katalysierte Acylierungsreaktion.

Schritt (iii) dient dazu, das in Schritt (ii) erhaltene Verhältnis der optischen Isomeren des 2-Methylalkan-1-ols (III) noch weiter zu verbessern. Dazu wird das in Schritt (ii) erhaltene Gemisch unter katalytischer Wirkung einer Lipase mit einem Acylierungsmittel umgesetzt (acyliert), wobei die Lipase selektiv ein Enantiomer (III) acyliert und das andere Enantiomer (III) unverändert lässt.

Bevorzugt acyliert man mittels Lipase dasjenige Enantiomer (III), das in Schritt (ii) im Enantiomerenunterschuss gebildet wurde und lässt somit das in Schritt (ii) im Enantiomerenüberschuss gebildete Produkt (III) durch Lipase unverändert. Dadurch, dass das im Unterschuss in Schritt (ii) gebildete Enantiomer durch Acylierung abreagiert, erhöht sich die optische Reinheit - definiert als Enantiomerenüberschuss - des im Schritt (ii) im Enantiomerenüberschuss gebildeten Enantiomers (III).

Das Acylierungsprodukt von (III) lässt sich vom nicht-acylierten Enantiomer (III) durch gängige Verfahren wie Chromatografie, Destillation oder Extraktion abtrennen.

Die in Schritt (iii) eingesetzten Lipasen können aus einer Vielzahl von Organismen, beispielsweise Säugetieren (Schwein), insbesondere aber von Mikroorganismen, stammen. Bevorzugte Lipasen sind solche, die aus der Spezies Pseudomonas oder Burkholderia stammen oder ausgehend von diesen durch gentechnische Verfahren verändert worden sind, besonders bevorzugt aus Burkholderia plantarii oder Pseudomonas fluorescens.

Besonders bevorzugt werden in Schritt (iii) die folgenden Lipasen verwendet: PFL Fluka (aus Pseudomonal fluorescens), Novo 435 (Novozymes), Amano PS-C1, Amano PS-C2 und Amano PS-D1 (aus Burkholderia cepacia, CAS -Nr 9001-62-1).

Die Lipasen können in gelöster Form oder auch in geträgerter Form eingesetzt werden. Bevorzugt verwendet wird eine Träger-gebundene Lipase

Als Acylierungsmittel für Schritt (iii) sind eine Vielzahl von Carboxylderivaten einsetzbar, die unter Lipase-Katalyse eine selektive Acylierung des Substrates (III) bewirken. Bevorzugte Acylierungsmittel sind Säureanhydride und Alkenylester.

Besonders bevorzugt sind cyclische Säureanhydride wie Bernsteinsäureanhydrid oder Vinylester.

Der Schritt (iii) kann mit oder ohne zusätzliches Lösungsmittel durchgeführt werden.

Wenn ein Lösungsmittel eingesetzt werden soll, sind folgende Lösungsmittel bevorzugt: CH₃CN, DMSO, NMP, CH₂Cl₂, CHCl₃, Dichlorethan, EtOAc, THF, TBME, Et₂O, Bu₂O, 1,4-Dioxan, Aceton, 2-Butanon, Toluen, Xylen, Benzen, Alkane der allgemeinen Formel CₙH₂ₙ₊₂ mit n = 5-15 und Mischungen davon.

Die bevorzugte Temperatur für diesen Schritt liegt zwischen 0-60°C, besonders bevorzugt zwischen 5-35°C.

Die Reaktionszeit kann je nach gewählten Bedingungen zwischen einer Stunde und mehreren Tagen betragen. Üblicherweise verfolgt man den Verlauf der Reaktion in Schritt (iii) und bricht die Reaktion dann ab, wenn der gewünschte Enantiomerenüberschuss erreicht ist.

Anschliessend trennt man das aus dem einen Enantiomer (III) gebildete Acylierungsprodukt vom anderen, nicht-umgesetzten Enantiomer (III) ab. Wenn als Acylierungsmittel ein Säureanhydrid eingesetzt worden ist, kann die Abtrennung des acylierten Produktes bevorzugt durch Extraktion mit wässriger Baselösung erfolgen.

Das erfindungsgemässe Verfahren kann sowohl in allen Schritte diskontinuierlich oder kontinuierlich betrieben werden; es ist aber auch möglich einzelne Schritte wie etwa Schritt (ii) kontinuierlich zu betreiben und die anderen Schritte diskontinuierlich.

### Experimenteller Teil

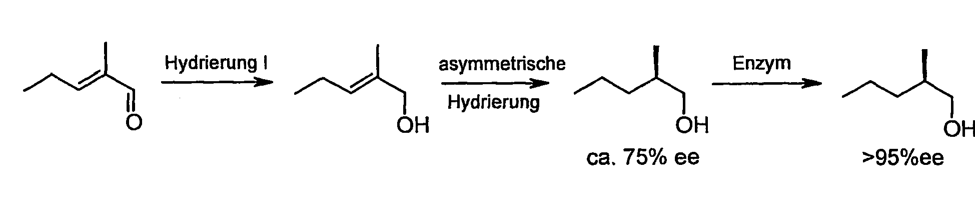

### Beispiel 1: Darstellung von 2-Methylpent-2-en-1-ol mit LiAlH₄

Zu einer Suspension von 745 mmol (28,28 g) Lithiumaluminumhydrid in 550ml Diethylether werden bei -78°C 595 mmol (58,4 g) 2-Methylpent-2-enal in 250 ml Diethylether getropft. Es wird 30 min bei 0°C und 1,5h bei Raumtemperatur gerührt, wieder auf 0°C abgekühlt und tropfenweise innerhalb von 40 min mit 50ml Wasser versetzt. Nach beendeter Zugabe werden 30 ml einer 13%igen Natronlauge und anschließend weitere 25 ml Wasser zugetropft. Die resultierende farblose Suspension wird über Celite-Filtergel filtriert, über MgSO₄ getrocknet und am Rotationsverdampfer bei Normaldruck eingeengt. Das Rohprodukt wird bei 11mbar und 52°C destilliert. Man erhält 467 mmol (46,8 g, 79% Ausbeute) 2-Methylpent-2-en-1-ol.

GC: Detektor: FID; Trennsäule: 25m * 0.32mm OV-1 (Macherey&Nagel); Filmdicke= 0.5 µm; Temperaturprogramm: 50°C, 2', 20°C/', 300°C: RT = 7,99 min 2-Methylpent-2-en-1-ol.

### Beispiel 2: Darstellung von 2-Methylpent-2-en-1-ol mit Ru/Fe/C-Kat.

Eine Mischung aus 349 mmol (35,0 g) 2-Methylpent-2-en-1-al, 17 ml MeOH, 1,5 g NMe₃ und 0,35 g BV 191 pass. wird bei 60°C und 40 bar Wasserstoffdruck für 22 h gerührt. Nach Abkühlen auf Raumtemperatur und Filtration erhält man ein Rohprodukt mit der folgenden Zusammensetzung (GC-Fl.%; ohne MeOH und NMe₃):
GC: Detektor: FID; Trennsäule 30m x 0,32mm Optima Wax (Macherey&Nagel); Filmdicke = 0,5 µm; Temperaturprogramm: 50°C, 2', 20°C/', 150°C, 15', 20°C/', 300°C;

| RT | Fl.% | Verbindung |
|---|---|---|
| 3,91 | 3,57 | 2-Methylpentanal |
| 4,21 | 0,34 | 2-Propylacrolein |
| 5,60 | 6,96 | 2-Methylpent-2-enal |
| 6,80 | 22,61 | 2-Methylpentanol |
| 7,59 | 63,4 | 2-Methylpent-2-en-1-ol |

### Beispiel 3: Hydrierung von 2-Methylpent-2-en-1-ol mit Ru/Josiphos-System

Zu einer Suspension von 13 µmol (4,2 mg) Ru(COD)(methallyl)₂ in 10 ml MeOH werden sukzessive 13 µmol (7,7 mg) Josiphos und 26 µmol (2,5 mg) Methansulfonsäure gegeben und für 30 min bei Raumtemperatur in der glove-box gerührt. Die resultierende Lösung wird zu einer Lösung von 65 mmol (6,51 g) 2-Methylpent-2-en-1-ol in 30 ml MeOH in einen Autoklaven unter Schutzgas gegeben. Es wird bei 25°C und 85 bar Wasserstoffdruck für 17 h gerührt. Das Rohprodukt hat die unten aufgeführte Zusammensetzung (GC-Fl.% ohne Me-OH) und einen ee von 58%

GC: Detektor: FID; Trennsäule 30m x 0,32mm Optima Wax (Macherey&Nagel); Filmdicke = 0,5 µm; Temperaturprogramm: 50°C, 2', 20°C/', 150°C, 15', 20°C/', 300°C;

| RT | Fl.% | Verbindung |
|---|---|---|
| 5,17 | 1,86 | 1,1'-Dimethoxy-2-methylpentan |

| | | |
|---|---|---|
| 7,12 | 97,82 | 2-Methylpentanol |
| 7,9 | 0,08 | 2-Methylpent-2-en-1-ol |

GC-Trennung R-, S- 2-Methylpentanol (4 Blindproben erforderlich): Säulenschaltung mit Vorsäule: 10m * 0.25mm Optima1 (Macherey&Nagel) FD=0,5microm, und Chirale Säule: 30m * 0.25mm BGB174 (BGB-Analytikvertrieb) FD=0,25microm; Ofentemp.: 60°C isotherm; Vorsäule: 0,3bar He, Chirale Säule: 1,1 bar He ; Säulenschaltung: RT= 2,9bis 3,5: Valve1=On ; RT=2,9min: Erhöhung des Vorsäulendruck auf 1,3bar für 0,3min : RT R-2-Methylpentanol = 1,9 min ; RT S-2-Methylpentanol = 4,1 min.

### Beispiel 4: Hydrierung von 2-Methylpent-2-en-1-ol mit Ru/Solphos-System

Zu einer Suspension von 13 µmol (4,2 mg) Ru(COD)(methallyl)₂ in 10 ml MeOH werden sukzessive 13 µmol (8,6 mg) R-Solphos und 26 µmol (2,5 mg) Methansulfonsäure gegeben und für 30 min bei Raumtemperatur in der glove-box gerührt. Die resultierende Lösung wird zu einer Lösung von 260 mmol (26,04 g) 2-Methylpent-2-en-1-ol in 120 ml MeOH in einen Autoklaven unter Schutzgas gegeben. Der Autoklave wird verschlossen, unter Stickstoff bei 280 bar abgepresst und in den vorgesehenen Stellplatz gebracht. Es wird bei 40°C und 200 bar Wasserstoffdruck für 17 h gerührt. Das Rohprodukt hat die unten aufgeführte Zusammensetzung (GC-FI.% ohne MeOH) und einen ee von 74%

| RT | FI.% | Verbindung |
|---|---|---|
| 4,63 | 1,66 | 1,1'-Dimethoxy-2-methylpentan |
| 6,52 | 79,87 | 2-Methylpentanol |
| 7,29 | 17,92 | 2-Methylpent-2-en-1-ol |

### Beispiel 5: Lipase katalysierte Acylierung

50 g (490 mmol) R-2-Methylpentanol mit 66 % ee wurden in 500 ml MTBE bei RT vorgelegt und mit 24,5 g(245 mmol) Bernsteinsäureanhydrid sowie 2,5 g Amano-lipase PS-D auf Clay versetzt und 2 d bei RT gerührt. Der Reaktionsfortgang wurde per GC verfolgt. Sobald der Ziel-ee-wert erreicht wird, wird das Enzym abfiltriert und das Filtrat zweimal mit jeweils 500 g 10 % Sodalösung ausgewaschen. Die vereinigten wässrigen Phasen wurden noch einmal mit 250 ml MTBE rückextrahiert; die vereinigten organischen Phasen wurden über MgSO₄ getrocknet, vom MTBE befreit und der Rückstand fraktioniert destilliert. Es wurden 14,5 g (35 % d. Th.) R-2-Methylpentanol mit 97 % ee und > 98 % chem. Reinheit als farbloses Öl erhalten.

### Beispiel 6: Lipase katalysierte Acylierung

50 g (490 mmol) R-2-Methylpentanol mit 66 % ee wurden in 200 ml THF bei RT vorgelegt und mit 24,5 g(245 mmol) Bernsteinsäureanhydrid sowie 0,5 g Amano-lipase PS-D auf Clay versetzt und 2 h bei RT gerührt. Der Reaktionsfortgang wurde per GC verfolgt. Sobald der Ziel-ee-wert erreicht wird, wird das Enzym abfiltriert und das Filtrat nach Zugabe von 100 ml MTBE zweimal mit jeweils 500 g 10 % Sodalösung ausgewaschen. Die vereinigten wässrigen Phasen wurden noch einmal mit 250 ml MTBE rückextrahiert; die vereinigten organischen Phasen wurden über MgSO₄ getrocknet, vom MTBE befreit und der Rückstand fraktioniert destilliert.
Es wurden 25,1 g R-2-Methylpentanol mit 97 % ee und > 98 % chem. Reinheit als farbloses Öl erhalten.

## Patentansprüche

1. Verfahren zur Herstellung von optisch aktivem 2-Methylalkan-1-ol der allgemeinen Formel (III) umfassend folgende Schritte:
(i) carbonylselektive Reduktion von 2-Methylalk-2-en-1-al der allgemeinen Formel (I) zu 2-Methylalk-2-en-1-ol der allgemeinen Formel (II),
(ii) enantioselektive Hydrierung von 2-Methylalk-2-en-1-ol zu der allgemeinen Formel (iii),
(iii) Erhöhung der optischen Ausbeute des in Schritt (ii) erhaltenen optisch aktiven 2-Methylalkan-1-ol (III) durch eine Lipase-katalysierte Acylierungsreaktion, 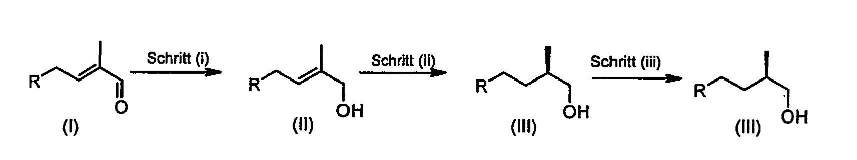
wobei der Rest R die Bedeutung C₁-C₁₀-Alkyl besitzt.

2. Verfahren nach Anspruch 1, **dadurch gekennzeichnet, dass** R die Bedeutung Methyl besitzt.

3. Verfahren nach Anspruch 1 und 2, **dadurch gekennzeichnet, dass** die Reduktion in Schritt (i) mittels eines komplexen Hauptgruppenelementhydrids durchgeführt wird.

4. Verfahren nach Anspruch 1 und 2, **dadurch gekennzeichnet, dass** die Reduktion in Schritt (i) mittels eines heterogenen Hydrierkatalysators durchgeführt wird.

5. Verfahren nach Anspruch 1 und 2, **dadurch gekennzeichnet, dass** die Hydrierung in Schritt (ii) mittels eines Übergangsmetallkomplexkatalysators durchgeführt wird.

6. Verfahren nach Anspruch 5, **dadurch gekennzeichnet, dass** als Übergangsmetall Ru, Rh, Ir, Pd oder Pt verwendet wird.

7. Verfahren nach Anspruch 5, **dadurch gekennzeichnet, dass** als Ligand des Übergangsmetallkomplexkatalysators eine Phophor enthaltende chirale Verbindung eingesetzt wird.

8. Verfahren nach Anspruch 7, **dadurch gekennzeichnet, dass** als Ligand eine phosphorhaltige Verbindung mit der Fähigkeit Atropisomerie bezüglich zweier Aryl- bzw. Hetarylsysteme auszubilden eingesetzt werden, gemäß der folgenden Strukturen 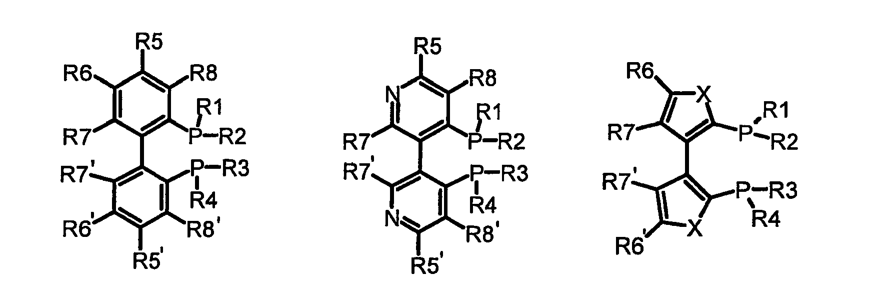 wobei:
R1, R2, die gleich oder verschieden sein können: subst. oder unsubst. Aryl, Heteroaryl, Alkyl, Cycloalkyl sein können,
R3, R4, die gleich oder verschieden sein können: subst. oder unsubst. Aryl, Heteroaryl Alkyl, Cycloalkyl sein können,
R5, R5', R6, R6', die gleich oder verschieden sein können: H, Halogen, Alkyl, Aryl, Alkoxy, Amino, Thio sein können,
R7, R7', die gleich oder verschieden sein können: H, Halogen, Alkyl, Aryl, Alkoxy, Amino, Thio sein können,
oder: R5, R5', die gleich oder verschieden sein können: H, Halogen, Alkyl, Aryl, Alkoxy, Amino, Thio
und R6 und R7, R6' und R7' bilden einen oder mehrere Ringe, die weitere 1 oder 2 Doppelbindungen enthalten können und/oder Heteroatome enthalten können,
R8, R8', die gleich oder verschieden sein können: H, Halogen, Alkyl, Aryl, Alkoxy, Amino, Thio sein können,
X, X', die gleich oder verschieden sein können: S, O, NR9
R9 = H, Alkyl, Aryl, Acyl,
SO₂R10
R₁₀ = Aryl, Alkyl,
Fluoroalkyl, CF₃

9. Verfahren nach Anspruch 7, **dadurch gekennzeichnet, dass** als Ligand eine der folgenden Verbindungen eingesetzt werden: (R)-2,2'-Bis-diphenylphosphanyl-[1,1']binaphthalenyl; (S)-2,2'-Bis-diphenylphosphanyl-[1,1']binaphthalenyl; (R)-6,6'-Bis-diphenylphosphanyl-2,3,2',3'-tetrahydro-[5,5']bi[benzo[1,4]dioxinyl]; (S)-6,6'-Bis-diphenylphosphanyl-2,3,2',3'-tetrahydro-[5,5']bi[benzo[1,4]dioxinyl]; (R)-7,7'-Bis-diphenylphosphanyl-4,4'-dimethyl-3,4,3',4'-tetrahydro-2*H*,2'*H*-8,8']bi[benzo[1,4]oxazinyl]; (S)-7,7'-Bis-diphenylphosphanyl-4,4'-dimethyl-3,4,3',4'-tetrahydro-2*H*,2'*H-*[8,8']bi[benzo[1,4]oxazinyl]

10. Verfahren nach Anspruch 1, **dadurch gekennzeichnet, dass** als Lipase in Schritt (iii) Amano Lipase PS-D1 (CAS-Nr. 9001-62-1), PS-C1 oder PS-C2 eingesetzt wird.

11. Verfahren nach Anspruch 1, **dadurch gekennzeichnet, dass** als Acylierungsmittel in Schritt (iii) Bernsteinsäureanhydrid verwendet wird.

12. Verfahren nach Anspruch 1, **dadurch gekennzeichnet, dass** in Schritt (iii) als Lösungsmittel THF,MTBE, Diethylether, Toluol oder 1,4-Dioxan verwendet wird.

13. Verfahren nach Anspruch 1, **dadurch gekennzeichnet, dass** im Anschluss an Schritt (i) eine Destillation zur Reinigung des erhaltenen 2-Methylalk-2-en-1-ol (II) durchgeführt wird.

## Claims

1. A process for preparing optically active 2-methylalkan-1-ol of the general formula (III) comprising the following steps:
(i) carbonyl-selective reduction of 2-methylalk-2-en-1-al of the general formula (I) to 2-methylalk-2-en-1-ol of the general formula (II),
(ii) enantioselective hydrogenation of 2-methylalk-2-en-1-ol to the general formula (iii),
(iii) increasing the optical yield of the optically active 2-methylalkan-1-ol (III) obtained in step (ii) by a lipase-catalyzed acylation reaction, 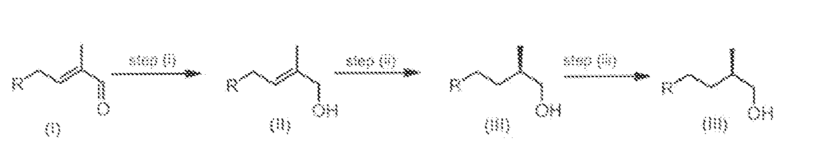
where the radical R means C₁-C₁₀-alkyl

2. The process according to claim 1, wherein R means methyl.

3. The process according to claim 1 and 2, wherein the reduction in step (i) is carried out using a complex-main group element hydride.

4. The process according to claim 1 and 2, wherein the reduction in step (i) is carried out using a hetorogeneous hydrogenation catalyst.

5. The process according to claim 1 and 2, wherein the hydrogenation in step (ii) is carried out using a transition metal complex catalyst.

6. The process according to claim 5, wherein Ru, Rh, Ir, Pd or Pt is used as transition metal.

7. The process according to claim 5, wherein a phosphorus-comprising chiral compound is employed as ligand of the transition metal complex.

8. The process according to claim 7, wherein a phosphorus-containing compound with the ability to develop atropisomerism in relation to two aryl or hetaryl systems are employed as ligang, according to the following structures 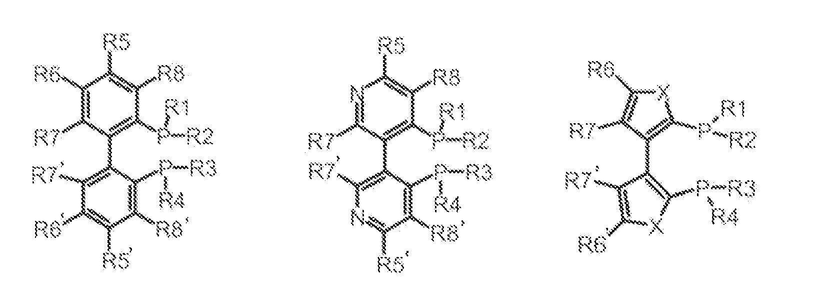 where:
R1, R2, which may be identical or different: may be subst, or unsubst. aryl, heteroaryl, alkyl, cycloalkyl,
R3, R4, which may be identical or different: may be subst. or unsubst. aryl, heteroaryl, alkyl, cycloalkyl,
R5, R5', R6, R6', which may be identical or different: may be H, halogen, alkyl, aryl, alkoxy, amino, thio,
R7, R7', which may be identical or different: may be H, halogen, alkyl, aryl, alkoxy, amino, thio,
or: R5, R5', which may be identical or different: may be H, halogen, alkyl, aryl, alkoxy, amino, thio
and R6 and R7, R6' and R7' form one or more rings which may comprise a further 1 or 2 double bonds and/or may comprise heteroatoms,
R8, R8', which may be identical or different: may be H, halogen, alkyl, aryl, alkoxy, amino, thio,
X, X', which may be identical or different: may be S, O, NR9
R9 = H, alkyl, aryl, acyl, SO₂R10
R10 = aryl, alkyl, fluoroalkyl, CF₃

9. The process according to claim 7, wherein one of the following compounds are employed as ligand: (R)-2,2'-bis-diphenylphosphanyl-[1,1]binaphthalenyl; (S)-2,2'-bis-diphenylphosphanyl-[1,1]binaphthalenyl; (R)-6,6'-bis-diphenylphosphanyl-2,3,2',3'-tetrahydro-[5,5']bi[benzo[1,4]dioxinyl]; (S)-6,6'-bis-diphenylphosphanyl-2,3,2',3'-tetrahydro-[5,5']bi[benzo[1,4]dioxinyl];(R)-7,7'-bis-diphenylphosphenyl-4,4'-dimethyl-3,4,3',4'-tetrahydro-2*H*,2'*H*-8,8']bi[benzo[1,4]oxazinyl; (S)-7,7'-bis-diphenylphosphanyl-4,4'-dimethyl-3,4,3',4'-tetrahydro-2*H*,2'*H-*[8,8']bi[benzo[1,4]oxazinyl].

10. The process according to claim 1, wherein Amano lipase PS-D1 (CAS No. 9001-62-1), PS-C1 or PS-C2 is employed as lipase in step (iii),

11. The process according to claim 1, wherein succinic anhydride is used as acylating agent in step (iii).

12. The process according to claim 1, wherein THF, MTBE, diethyl ether, toluene or 1,4-dioxane is used as solvent in step (iii).

13. The process according to claim 1, wherein a distillation is carried out to purify the 2-methylalk-2-en-1-ol (II) obtained following step (i).

## Revendications

1. Procédé de préparation de 2-méthylalcan-1-ol optiquement actif de formule générale (III), comportant les étapes consistant à :
(i) réduire de manière sélective le groupe carbonyle du 2-méthylalc-2-én-1-al de formule générale (I) pour donner du 2-méthylalc-2-én-1-ol de formule générale (II),
(ii) hydrogéner de manière énantiosélective le 2-méthylalc-2-én-1-ol pour donner la formule générale (III),
(iii) augmenter le rendement optique du 2-méthylalcan-1-ol (III) optiquement actif obtenu dans l'étape (ii) selon une réaction d'acylation catalysée par une lipase, 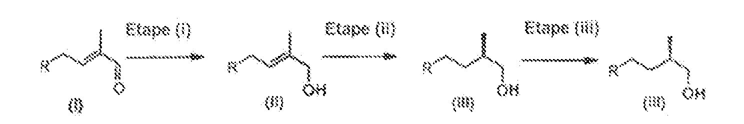
dans lesquelles le radical R signifie un groupe alkyle en C1-C₁₀.

2. Procédé selon la revendication 1, **caractérisé en ce que** R signifie un groupe méthyle.

3. Procédé selon les revendications 1 et 2, **caractérisé en ce que** la réduction est mise en oeuvre dans l'étape (1) à l'aide d'un hydrure complexe d'un élément du groupe principal.

4. Procédé selon les revendications 1 et 2, **caractérisé en ce que** la réduction est mise en oeuvre dans l'étape (i) à l'aide d'un catalyseur d'hydrogénation hétérogène.

5. Procédé selon les revendications 1 et 2, **caractérisé en ce que** l'hydrogénation est mise en oeuvre dans l'étape (ii) à l'aide d'un catalyseur constitué d'un complexe de métal de transition.

6. Procédé selon, la revendication 5, **caractérisé en ce que** Ru, Rh, Ir, Pd ou Pt est utilisé en tant que métal de transition.

7. Procédé selon la revendication 5, **caractérisé en ce qu'**un composé chiral contenant du phosphore est utilisé en tant que ligand du catalyseur constitué d'un complexe de métal de transition.

8. Procédé selon la revendication 7, **caractérisé en ce qu'**un composé contenant du phosphore et ayant la faculté de former une atropoisomérie par rapport à deux systèmes aryle ou bien hétaryle est utilisé en tant que ligand, en se conformant aux structures suivantes : 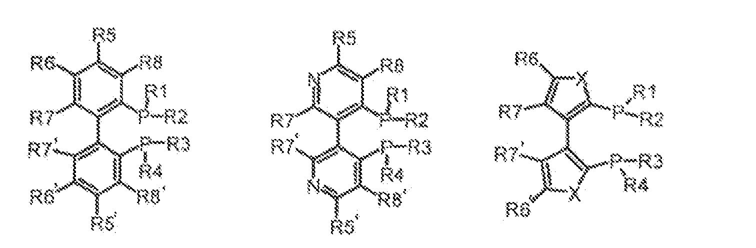 dans lesquelles:
R1, R2, qui peuvent être identiques ou différents ; peuvent être un groupe aryle, hétéroaryle, alkyle, cycloalkyle substitué ou non substitué,
R3, R4, qui peuvent être identiques ou différents ; peuvent être un groupe aryle, hétéroaryle, alkyle, cycloalkyle substitué ou non substitué,
R5, R5', R6, R6', qui peuvent être identiques ou différents ; peuvent être H, un groupe halogène, alkyle, aryle, alcoxy, amino, thio,
R7, R7', qui peuvent être identiques ou différents ; peuvent être H, un groupe halogène, alkyle, aryle, alcoxy, amino, thio,
ou : R5, R5', qui peuvent être identiques ou différents : H, un groupe halogène, alkyle, aryle, alcoxy, amino, thio,
et R6 et R7, R6' et R7' forment un ou plusieurs cycles, qui peuvent contenir d'autres 1 ou 2 doubles liaisons et/ou peuvent contenir des hétéroatomes,
R8, R8', qui peuvent être identiques ou différents : peuvent être H, un groupe halogène, alkyle, aryle, alcoxy, amino, thio,
X, X', qui peuvent être identiques ou différents :
S, O, NR9
R9 = H, alkyle, aryle, acyle, SO₂R10
R10 = aryle, alkyle, fluoroalkyle, CF₃.

9. Procédé selon la revendication 7, **caractérisé en ce qu'**un des composés suivants est utilisé en tant que ligand : (R)-2,2'-bis-diphénylphosphanyl-[1,1']-binaphtalényle ; (S)-2,2'-bis-diphénylphosphanyl-[1,1']binaphtalényle ; (R)-6,6'-bis-diphénylphosphanyl-2,3,2',3'-tétrahydro-[5,5']bi[benzo[1,4]-dioxinyle] ; (S)-6,6'-bis-diphénylphosphanyl-2,3,2',3'-tétrahydro-[5,5'] bi[benzo[1,4]dioxinyle] ; (R)-7,7'-bis-diphénylphosphanyl-4,4'-diméthyl-3,4,3',4'-tétrahydro-2*H*,2'*H*-[8,8']bi[benzo-[1,4]oxazinole] ; (S)-7,7'-bis-diphénylphosphanyl-4,4'-diméthyl-3,4,3',4'-tétrahydro-2*H*,2'*H*-[8,8']bibenzo[1,4]oxazinyle].

10. Procédé selon la revendication 1, **caractérisé en ce que** de la lipase amano PS-D1 (CAS - n° 9001-62-1), PS-C1 ou PS-C2 est utilisée en tant que lipase dans l'étape (iii).

11. Procédé selon la revendication 1, **caractérisé en ce que** de l'anhydride d'acide succinique est utilisé en tant qu'agent d'acylation dans l'étape (iii).

12. Procédé selon la revendication 1, **caractérisé en ce que** du THF, du MTBE, du diéthyléther, du toluène ou du 1,4-dioxanne est utilisé en tant que solvant dans l'étape (iii).

13. Procédé selon la revendication 1, **caractérisé en ce qu'**une distillation destinée à purifier le 2-méthylalc-2-én-1-ol (II) obtenu est mise en oeuvre à la suite de l'étape (i).
